# FASCICULE DE BREVET EUROPEEN

(11) **EP 0 375 767 B1**
(45) Date de publication et mention de la délivrance du brevet: **20.12.1995**
(21) Numéro de dépôt: 89907183.1
(22) Date de dépôt: 06.06.1989
(51) Int. Cl.: C07K 4/02, C07K 14/005, C07K 14/16, A61K 38/02, A61K 38/03, A61K 39/21, C12Q 1/70

(54) **PEPTIDES AYANT DES PROPRIETES PROTECTRICES D'UN VIRUS PATHOGENE DU TYPE HIV DANS DES CELLULES SENSIBLES**
PEPTIDE MIT SCHUTZEIGENSCHAFTEN GEGEN EIN PATHOGENES VIRUS DES HIV-TYPS IN SENSITIVEN ZELLEN
PEPTIDES WITH PROTECTIVE PROPERTIES WITH REGARD TO A PATHOGENIC VIRUS OF THE HIV TYPE IN SENSITIVE CELLS

(30) Priorité: 06.06.1988 FR 8807504
(43) Date de publication de la demande: 04.07.1990
(73) Titulaire: INSTITUT PASTEUR, 75724 Paris Cédex 15 (FR); INSTITUT NATIONAL DE LA SANTE ET DE LA RECHERCHE MEDICALE (INSERM), F-75654 Paris Cédex 13 (FR)
(72) Inventeur: CLAVERIE, Jean-Michel, F-75014 Paris (FR); BOUGELERET, Lydie, F-75015 Paris (FR); KOURILSKY, Philippe, F-75001 Paris (FR); LANGLADE-DEMOYEN, Pierre, F-75015 Paris (FR); CHALUFOUR-PROCHNICKA, Ada, F-92120 Montrouge (FR); DADAGLIO, Gilles, F-75020 Paris (FR); TEKAIA, Fredj, F-77100 Meaux (FR); PLATA, Fernando, F-75006 Paris (FR)
(74) Mandataire: Gutmann, Ernest
(86) Numéro de dépôt international: FR8900285
(87) Numéro de publication internationale: WO8912066

(56) Documents cités:
- EP-A- 0 230 222
- EP-A- 0 267 802
- WO-A-86/02383
- WO-A-86/04336
- WO-A-87/07958
- FR-A- 2 603 107
- GB-A- 2 188 639
- Proc. Natl. Acad. Sci, USA, Vol. 84, Mai 1987 P. KOURILSKY et al.: "Working Principles in the Immune System Implied by the "Peptide Self" Model", pages 3400-3404

## Description

L'invention concerne des peptides ayant des propriétés lytiques à l'égard de cellules infectées par un virus HIV et les applications de ces peptides à la prévention de la prolifération de l'infection rétrovirale dans une population cellulaire sensible à l'infection, notamment des lymphocytes T4, en culture ou in vivo.

L'invention s'inscrit dans le développement de techniques qui ont fortement contribué à une nouvelle élaboration de modèles expliquant les mécanismes que le système immunitaire met en oeuvre pour organiser dans l'organisme d'un hôte la résistance à des agressions par des antigènes étrangers pathogènes.

Les résultats récents de plusieurs laboratoires (publications (1) à (6) dans la bibliographie à la fin de ce texte) convergent dans le sens d'une réévaluation du contexte dans lequel inter-agissent les lymphocytes, plus particulièrement les lymphocytes T et un antigène extérieur comportant une structure peptidique ou consistant en une protéine. Après dégradation intracellulaire de l'antigène pour produire des peptides courts, ceux-ci sont fixés à des molécules de classe I ou de classe II du complexe majeur d'histocompatibilité (CMH). Les complexes que forment alors ces peptides avec ces molécules sont ensuite reconnus par les récepteurs appropriés de cellules T. Le mécanisme de reconnaissance des antigènes par restriction-CMH fait l'objet de publications 7-9, revues dans (5), et le processus de transformation de l'antigène, celui de publications (6) et (10). Il a été montré récemment que ces mécanismes faisaient intervenir la fixation d'un peptide porteur d'un épitope-T initialement contenu dans l'antigène concerné à des molécules de CMH de classe II (publications (11) et (12)) et que l'on pouvait mettre en évidence une corrélation entre l'affinité de fixation in vitro et les systèmes de restriction et de reconnaissance fonctionnelle par des cellules auxiliaires T (publications (11), (13)-(15)). Les résultats d'autres équipes (16-19) soutiennent l'hypothèse que des molécules CMH de classe 1 présentent des peptides sélectionnés dérivés des protéines ou polypeptides antigéniques aux cellules T, dans des conditions semblables. L'hypothèse a été formulée que les récepteurs des cellules T reconnaissaient spécifiquement les peptides sélectionnés, à l'exclusion des régions polymorphes des molécules du CMH qui le présentent.

Une synthèse de ces hypothèses a été proposée dans une publication de P. KOURILSKY et al parue dans MEDECINE/SCIENCE 1988, n° 4:177-183.

Dans ce cadre général certaines règles ont été proposées pour identifier des épitopes T contenus dans des protéines (22-24). Ces règles présument de l'existence d'un certain nombre de contraintes auxquelles doit satisfaire la fixation d'un peptide issu d'un éventuel antigène sur la molécule CMH qui ensuite en assure la présentation. L'hypothèse a d'ailleurs été formulée que toutes les protéines étrangères à l'organismes de l'hôte et des protéines de l'hôte lui-même étaient présentées au terme du susdit processus de dégradation. Il a été proposé, en particulier dans la demande de brevet France n° 86.08896/2.600.335 déposée le 19 février 1986 -- et les travaux qui ont suivi tendent à confirmer l'hypothèse alors formulée -- que l'antigénicité d'une protéine étrangère pouvait être due au fait qu'elle donnait naissance, au terme du processus rappelé ci-dessus, à des peptides que le système immunitaire n'avait pas, jusque là, appris à reconnaître comme appartenant au "moi somatique" de l'hôte ou encore au "soi somatique" ou "soi peptidique", selon les différentes expressions qui ont été utilisées pour désigner le répertoire en séquences peptidiques de protéines de l'hôte. Les peptides dont les séquences sont érangères au "soi peptidique" formaient alors (ou en faisaient partie) l'épitope ou les épitopes porteurs de l'immunogénicité propre à déclencher les processus immunitaires de défense.

Cette demande de brevet proposait aussi un procédé pour l'analyse, au sein d'une protéine étrangère à l'organisme d'un hôte appartenant à une espèce déterminée de la séquence peptidique portant l'épitope responsable de l'immunogénicité, ce procédé étant alors caractérisé par la combinaison d'étapes que constituent :
- la détermination et l'identification de la famille des oligopeptides ayant une longueur déterminée, par exemple des tétrapeptides, qui sont contenus dans la séquence en acides aminés constitutifs de cette protéine étrangère,
- la comparaison de chacun des tétrapeptides de cette famille avec chacun des tétrapeptides de l'ensemble des tétrapeptides contenus dans les protéines dont les séquences en acides aminés sont connues et qui sont disponibles dans un catalogue pré-établi de ces protéines, toutes ces protéines étant caractéristiques du soi somatique de l'espèce correspondante,
- l'identification de ceux des tétrapeptides (tétrapeptides non coïncidents) de la famille de la protéine étrangère pour lesquels n'existent que peu de coïncidences et, de préférence, aucune coïncidence avec les tétrapeptides disponibles dans le catalogue,
- la recherche, au sein de la protéine étrangère de séquences formées de tétrapeptides non coïncidents qui se chevauchent mutuellement, et
- l'analyse parmi ces séquences de tétrapeptides non coincidents et se chevauchant d'au moins une séquence d'acides aminés contenue dans l'épitope recherché.

Le "catalogue", dont il a également été question plus haut, correspond à toutes banques de données dans lesquelles sont répertoriées les séquences en acides aminés de protéines en nombre de plus en plus important. L'expression s'applique également aux banques de données dans lesquelles sont rassemblées toutes les informations relatives aux séquences nucléotidiques des fragments d'ADNs séquencés, parmi lesquels de nombreux ADNs codant pour des protéines dont les séquences en acides aminés peuvent alors être déduites au moyen du code génétique.

On comprendra que toute protéine formée d'un nombre x d'aminocides contient x-3 tétrapeptides, respectivement décalés d'un acide aminé les uns par rapport aux autres selon la définition rappelée ci-dessus. Si l'on part de l'extrémité N-terminale de la protéine, le premier des tétrapeptides du genre en question est formé à partir des quatre premiers aminoacides à compter de l'extrémité N terminale, le second oligopeptide ou tétrapeptide s'étendra entre le deuxième et le cinquième acide aminé de la séquence de la protéine totale, etc. et le dernier du (x-3)^{ème} à l'aminoacide C-terminal. Les "x-3" tétrapeptides successifs contenus dans cette protéine se chevauchent donc respectivement au niveau de séquences tripeptidiques qui, elles aussi, vont se succéder dans la structure de la protéine : par exemple tripeptide comprenant les 2ème, 3ème et 4ème acides aminés dans les premier et deuxième tétrapeptides à partir de l'extrémité N-terminale de la protéine ; le tripeptide suivant comprenant les 3ème, 4ème et 5ème acides aminés à partir de cette même extrémité N-terminale dans les 2ème et 3ème tétrapeptides et ainsi de suite, jusqu'au (x-3)ème tétrapeptide qui chevauche le (x-4)ème au niveau d'un tripeptide comprenant lui-même les (x-3)ème, (x-2)ème et (x-1)ème acides aminés de la protéine. Il sera encore dit dans la suite de cet exposé que ces tétrapeptides successifs sont respectivement décalés d'un acide aminé, les uns par rapport aux autres.

Partant de ces données, il a été remarquable de constater en rapport avec certains polypeptides de synthèse dont avait déjà été montrée la capacité d'induire in vivo des anticorps reconnaissant la protéine entière correspondante, que ces polypeptides se sont finalement avérés contenir des oligopeptides, plus particulièrement des tétrapeptides, qui n'avaient pas leurs équivalents au sein des ensembles des familles d'oligopeptides contenues dans les protéines, dont les séquences en acides aminés sont disponibles dans les banques de données qui avaient été utilisées en rapport avec les exemples décrits.

Il a même été constaté que cette absence de communauté entre oligopeptides de la protéine antigénique envisagée et oligopeptides, notamment tétrapeptides, des protéines répertoriées dans la banque de données s'étendait souvent à des oligopeptides successifs se chevauchant mutuellement, c'est-à-dire étant décalés dans la séquence de la protéine chaque fois d'un aminoacide. Cette constatation donne donc un fondement raisonnable à l'hypothèse que les séquences peptidiques "non coïncidentes" correspondantes sont, à l'occasion de la fragmentation intracellulaire au hasard de la protéine antigénique, reconnues comme étrangères au "soi somatique" par le système immunitaire de l'hôte, ce mécanisme de reconnaissance étant alors à l'origine du déclenchement des processus de défense en vue de la destruction de la protéine antigènique in vivo.

Le choix de tétrapeptides pour la réalisation des essais qui avaient été décrits plutôt que d'oligopeptides yant des nombres distincts d'acides aminés avait été dicté par l'étendue des informations disponibles à l'époque quant aux séquences en acides aminés de protéines appartenant à une espèce donnée. On mentionnera par exemple que les totaux des acides aminés contenus dans les séquences des protéines de souris répertoriées, en 1986, dans la banque utilisée étaient de 26.000 pour les protéines "somatiques" de la souris et de 16.000 pour les protéines "somatiques du lapin. A ce jour, la validité des hypothèses faites en 1986 conserve toute son actualité.

En effet les constatations faites à l'époque ne sont pas invalidées pour l'essentiel par l'examen, dans les conditions évoquées dans la demande de brevet français des tétrapeptides aujourd'hui disponibles dans la banque de données NBRF-PIR (28) : 97.415 résidus pour l'homme et 28.168 pour la souris, soit 60,08 % et 17,6 %, respectivement, du nombre total des tétrapeptides différents qui peuvent être construits en faisant appel à toutes les possibilités de combinaison quatre à quatre des 20 acides aminés naturels. En outre dans l'état actuel des catalogues, la totalité des tétrapeptides répertoriés comme faisant partie des protéines somatiques de l'homme représente 44,6 % de la totalité des 160.000 combinaisons possibles, quatre à quatre, des 20 acides aminés naturels. L'accroissement récent de 25 % des protéines du "soi somatique" humain, répertoriées dans le catalogue des protéines du "soi somatique" concerné s'est accompagné d'un accroissement seulement de 4,6 % du nombre de séquences tétrapeptidiques constitutives des protéines somatiques de l'homme. Aussi peut-on d'ores et déjà penser que le répertoire des protéines du "soi somatique" pour une espèce donnée de mammifère est en fait relativement limité et, au surplus, que ce répertoire n'est pas loin d'être complet pour les séquences tétrapeptiques contenues dans les protéines du "soi somatique" de l'homme.

Il avait également déjà été remarqué dans la susdite demande de brevet que les molécules du système immunitaire, particulièrement les anticorps et les récepteurs des cellules T, peuvent être dégradées en peptides présentés à la surface des cellules du système immunitaire, où ils jouent sans doute un rôle régulateur par le biais de leur intervention dans les interactions entre cellules. Ainsi, selon que les régions variables des immunoglobulines ou des récepteurs des cellules T produisent des peptides qui ressemblent ou non au "soi somatique", des signaux régulateurs de nature très différente peuvent être obtenus. Il avait été envisagé que l'emploi de peptides ou de polypeptides ainsi définis et synthétisés par voie chimique (ou par des techniques du génie génétique) est donc de nature à moduler l'équilibre du système, notamment par le biais des régulations dites idiotypiques.

En fait, il a bien été confirmé que des protéines variables du système immunitaire pouvaient elles-mêmes être immunogènes et par conséquent être considérées comme étant étrangères vis-à-vis du "soi somatique". En d'autres termes, les séquences tétrapeptidiques incluses dans les séquences des protéines impliquées dans le système immunitaire lui-même, par exemple les immunoglobulines, les récepteurs de cellules T, les molécules du CMH, etc. doivent être exclues du catalogue des peptides constituant le "soi somatique" (publications (1) et (25)).

L'invention résulte de la découverte de ce que certains peptides découlant de séquences peptidiques contenues dans les nucléoprotéines du virus, et plus particulièrement d'un rétrovirus, pouvaient, dès lors qu'ils ne faisaient pas partie du "soi somatique" tel que défini ci-dessus, faire obstacle à la capacité du virus correspondant à proliférer dans des cultures cellulaires sensibles à l'infection par ce virus.

Les peptides selon l'invention sont caractérisés par le fait qu'ils contiennent, en commun avec une nucléoprotéine d'un virus contre lequel une protection peut être recherchée, des séquences tétrapeptidiques successives, qui se chevauchent mutuellement en ce qu'elles sont décalées mutuellement d'un acide aminé les unes par rapport aux autres, ces séquences n'étant pas elles-mêmes pour l'essentiel, contenues ou susceptibles d'être répertoriées dans un catalogue de séquences tétrapeptidiques, appartenant elles-mêmes à des protéines caractéristiques du "soi somatique" ou du "soi immunologique".

L'invention concerne plus particulièrement des peptides comportant x acides aminés et contenant une séquence en acides aminés en commun avec une nucléoprotéine d'un virus contre lequel une protection peut être recherchée, notamment avec la protéine gag d'un rétrovirus HIV, ce peptide étant caractérisé par le fait que les (x-3) séquences tétrapeptidiques successives entrant dans sa constitution et qui se chevauchent mutuellement, et qui sont respectivement décalées d'un acide aminé les unes par rapport aux autres, ne sont pas elles-mêmes, pour l'essentiel, contenues ou susceptibles d'être répertoriées dans un catalogue de séquences tétrapeptidiques, appartenant elles-mêmes à des protéines caractéristiques du "soi somatique" ou du "soi immunologique" de l'homme.

On rappelle à toutes fins utiles que les rétrovirus du type HIV (abréviation de la désignation anglaise "Human Immunodeficiency Virus" ou "virus d'immunodéficience humaine) constituent les agents étiologiques du SIDA (abréviation de "Syndrome d'Immunodeficience acquise).

Différentes publications décrivent des séquences polypeptidiques de nucléoproteines de rétrovirus ou de fragments de celles-ci, contenant des séquences tétrapeptidiques de l'invention. Cependant, aucun de ces documents ne décrit des peptides ayant les mêmes caractéristiques que ceux de l'invention.

La demande de brevet européen n° 0230222 A1 décrit des peptides de longueur variable (entre 14 et 56 kdaltons) qui possèdent une portion immunologiquement active de la nucléoprotéine gag de HTLVIII. L'hypothèse avancée est que ces peptides pourraient présenter une activité vaccinante ou antigénique. Mais, rien n'est dit sur leur capacité à inhiber l'infection virale par un mécanisme tel que décrit plus haut comme à la base de l'effet des peptides de l'invention. En outre, un effet de peptides plus courts n'est pas suggéré, l'immunogénicité de petits peptides n'étant pas reconnue.

La demande de brevet européen n° 0267802 décrit des peptides utilisables pour rechercher la présence d'anticorps spécifiques de HIV dans le sang humain. Ces peptides, d'au moins 5 acides aminés de long, sont capables d'être reconnus par des anticorps contre les protéines pol ou gag. En aucun cas, ils ne peuvent avoir d'applications autre que diagnostiques.

D'autres publications décrivent des séquences protéiques ou glycoprotéiques du virus HIV potentiellement immunogènes.

C'est le cas notamment de la demande WO86/02383. Ces publications ont toutes en commun de caractériser les protéines virales à des fins diagnostiques et éventuellement vaccinantes.

Cependant, aucune publication n'a approché des séquences particulières contenant exclusivement des tétrapeptides chevauchant n'appartenant pas au "soi somatique" ou "peptidique".

De préférence les peptides selon l'invention comportent au moins 3, voire au moins 4 séquences tétrapeptidiques successives du type sus-indiqué, en particulier des séquences contenant des résidus lysine ou alanine, ou les deux à la fois.

Ces peptides peuvent contenir jusqu'à 100 acides aminés, bien qu'il n'y ait aucune raison de voir une limite supérieure dans ce nombre. Avantageusement, il comprend de 6 à 20, notamment de 8 à 10 amonoacides.

Les peptides tels que ci-dessous définis sont en effet capables de rendre les cellules infectées par le virus correspondant sensibles à une lyse destructrice, tant de la cellule que du virus en cours de réplication dans ces cellules et par conséquent de prévenir la prolifération de l'invention. Cette propriété des peptides selon l'invention peut être mise en évidence par le procédé qui fait lui-même partie de l'invention, ce procédé comprenant la mise en contact de cellules T-cytotoxiques ayant une spécificité correspondant au type de virus concerné, par exemple HIV et restreintes par une molécule HLA-A2, avec des cellules d'une lignée de cellules tumorales transformées de façon stable avec un gène HLA-A2 et la détection de la lyse desdites cellules tumorales, lorsque l'incubation est réalisée en présence dudit peptide. Cette lyse est par exemple constatée par la libération dans le milieu de culture d'un marqueur interne auparavant incorporé à ces cellules tumorales.

Dans ce qui précède, "HLA" est l'abréviation de l'expression anglaise "human-leucocytes-associated", normalement suivie du mot "antigène" en d'autres termes, un antigène associé avec des leucocytes humains.

L'utilisation de l'expression "pour l'essentiel", à propos de l'absence à tout le moins souhaitée, sinon nécessaire, des tétrapeptides dans les catalogues susdits a pour objectif de laisser une porte ouverte sur la nécessité possible d'avoir à exclure postérieurement du répertoire certaines des séquences tétrapeptidiques jugées à ce jour comme pouvant entrer dans la constitution des peptides selon l'invention, dès lors qu'elles s'avèreraient appartenir à des protéines caractéristiques du "soi somatique" ou du "soi immunologique", comme suite à l'identification des séquences en acides aminés d'autres protéines du "soi somatique" de l'hôte concerné et à l'incorporation de leurs séquences tétrapeptidiques aux catalogues ou banques de données du genre susdit.Il apparaîtra néanmoins déjà clairement à l'homme du métier que l'élimination desdites séquences tétrapeptidiques n'altèrera pas le caractère général de l'invention. Cette élimination relèverait en conséquence des compétences normales de l'homme du métier, puisqu'il saurait modifier en conséquence les séquences en acides aminés des peptides selon l'invention, voire en restreindre le choix, surtout quand l'étude de la séquence en acides aminés de la nucléoprotéine du virus concerné dans les conditions précédemment indiquées fait apparaître, comme on le constate dans ce qui suit, à propos de la séquence gag d'un rérovirus HIV-1, la possibilité de l'existence de plusieurs séquences peptidiques éventuellement susceptibles de répondre aux conditions qui ont été énoncées à propos de la définition générale des peptides selon l'invention.

La présence de certaines séquences tétrapeptidiques qui s'avèreraient appartenir quand même au "soi somatique" dans les peptides susceptibles d'être choisis dans les conditions indiquées plus haut peut d'ailleurs n'avoir qu'une portée limitée, dès lors qu'il peut être établi que ces peptides contiennent de nombreuses autres séquences tétrapeptidiques répondant bien en fait aux conditions qui ont été précédemment indiquées. Cela paraît de toute évidence être le cas pour les quatre peptides :
GNFLQSRPEPTAPPA (gag 3)
GHQAAMEMLKE (gag 5)
HAGPIAPGQMREPRG (H15G)
KEGHQMKDCTERQANF (K16F)
dont les séquences sont toutes dérivées de protéines gag d'un rétrovirus HIV-1 et qui s'avèrent toutes capables de favoriser une lyse de cellules tumorales transformées de façon stable avec un gène HLA-A2, lorsque celles-ci sont incubées en leur présence et au contact de cellules T-cytotoxiques restreintes par une molécule HLA-A2 et ayant une spécificité HIV-1.

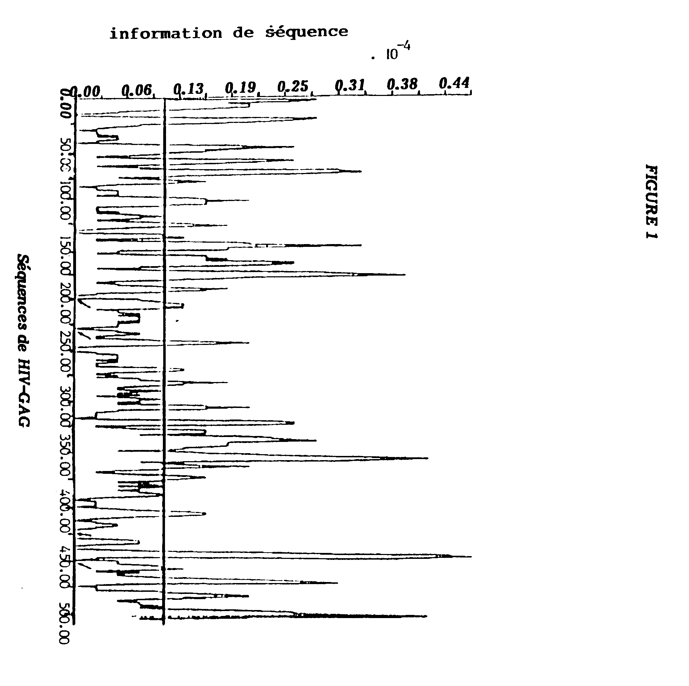

Des caractéristiques supplémentaires de l'invention apparaîtront encore à l'occasion de la description qui, dans ce qui suit, est donnée à titre d'exemple, des conditions dans lesquelles des peptides conformes à l'invention peuvent être identifiés et testés, à partir de régions très conservées au sein de nucléoprotéines gag de plusieurs isolats de rétrovirus HIV-1 (BRU, MAL, ARV-2 et ELI) (26). Référence est faite aux dessins dans lesquels : - la fig. 1 est une représentation graphique du profil des fréquences (axes des ordonnées) de présence des séquences tétrapeptidiques successives (axe des abscisses) de la protéine gag de HIV-1 (isolat BRU) dans un catalogue ou banque contenant les données choisies comme il est indiqué plus loin et dans les conditions évoquées dans la susdite demande de brevet français, pour ce qui est de la représentation graphique :

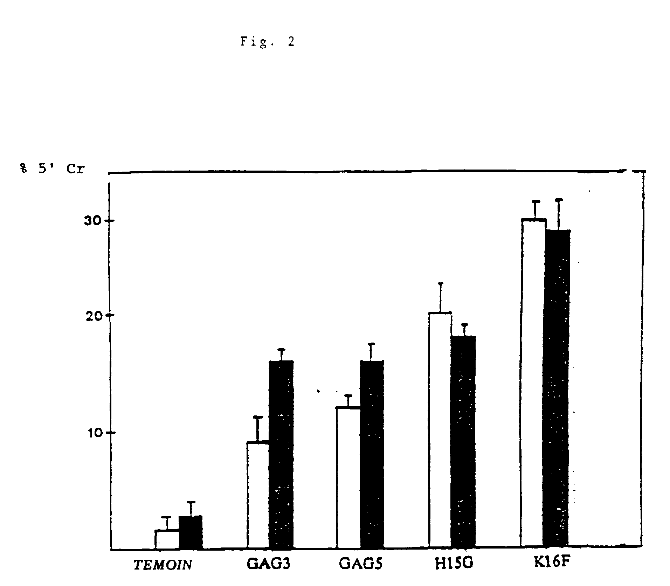

- la fig. 2 est représentative des degrés de lyse observés sur des cellules tumorales dans des essais de cytotoxicité dont le principe a été indiqué plus haut, en présence des 4 peptides identifiés plus haut.

L'examen de la fig. 2 fait apparaître la présence de 15 régions de la protéine gag de HIV-1 contenant des séquences tétrapeptidiques absentes du catalogue des protéines du "soi somatique" utilisé. L'attention des inventeurs s'est portée sur celles de ces régions :
- qui se trouvent être situées dans des régions particulièrement bien conservées de la nucléoprotéine des différentes variétés des HIV,
- qui comprennent au moins 5 séquences chevauchantes successives non coïncidentes avec les protéines du "moi somatique" et
- qui contiennent davantage de résidus lysine ou alanine ou des deux à la fois que les autres séquences :
d'où le choix qui a été effectué des séquences 193-203 (gag 5) ; 219-233 (H15G) ; 446-460 (gag 3) et 418-433 (K16F) de la protéine gag de HIV. Ce sont ces peptides qui ont été synthétisés et qui ont fait l'objet des essais décrits ci-après. Il convient néanmoins de relever au préalable que les paramètres qui ont guidé les choix précédents ont été tirés des études théoriques que les inventeurs ont menées et dont il est résulté que les épitopes immunogènes T dont l'existence avait été reconnue dans un nombre important de protéines antérieurement décrites étaient souvent caractérisables par au moins 3, voire même au moins 4 séquences tétrapeptidiques chevauchantes successives et non coïncidentes et par une proportion plus grande de résidus alanine et lysine -- même si ce critère ne peut être considéré à lui seul comme décisif -- que dans d'autres régions de la nucléoprotéine du virus.

L'activité des peptides selon l'invention a été mise en évidence dans un essai de cytotoxicité réalisé dans les conditions suivantes. Une lignée de cellules T cytotoxiques spécifiques de HIV, restreintes par HLA-A2, a été produite à partir de lymphocytes sanguins humains périphériques par stimulation avec des lymphoblastes infectés avec un HIV autologue et irradiés par des rayons X (C10.000 Rads) en présence d'interleukine-2. La capacité des peptides à reconnaître les cellules T-cytotoxiques dérivées des susdits lymphocytes (cellules CTL) a été testée par mise en contact de ces cellules T-cytotoxiques, en présence de doses variables desdits peptides, avec des cellules tumorales cibles issues d'une tumeur de souris P815, transformées de façon stable par un gène HLA-A2 (31) et marquées par du ⁵¹Cr, dans un rapport de 2 cellules cytotoxiques pour 1 cellule cible, et ce dans un essai classique de cytotoxicité ((27), (32)). Les essais ont été réalisés en triple. La cytotoxicité spécifique a été déterminée au terme d'une incubation pendant 15 heures. L'activité lytique spécifiquement induite par les peptides selon l'invention a été appréciée par l'accroissement du pourcentage de libération dans le milieu du marqueur ⁵¹Cr, par rapport à la quantité de marqueur libéré spontanément, en l'absencce des inducteurs dans les conditions expérimentales choisies : 14 à 18 % de la quantité totale de marqueur initialement incorporé. Les accroissements des pourcentages relatifs de marqueur libéré (% ⁵¹Cr sur l'axe des ordonnées de la fig. 2) par rapport à une expérience témoin effectuée dans les mêmes conditions, mais en présence d'un peptide réputé dépourvu de tout épitope du type immunogène T (RKRSQMLFRGRRASQ), résultent des hauteurs relatives des barres verticales apparaissant dans cette même figure. Les barres blanches correspondent à des doses des peptides indiqués sous l'axe des abscisses de 3 microgrammes/millilitre de milieu, les barres noires à des doses de 12 microgrammes/millilitre.

L'examen de la fig. 2 fait donc apparaître l'importante capacité des peptides selon l'invention de promouvoir la lyse des cellules infectées par le rétrovirus HIV. En conséquence de quoi l'invention fournit un nouveau principe actif susceptible de protéger une population de cellules infectables par le virus HIV, si bien que l'utilisation de ces peptides (en association avec des excipients pharmaceutiquement acceptables) par la constitution de compositions pharmaceutiques pour le traitement in vivo peut être sérieusement envisagée. En tout état de cause, l'invention fournit un moyen permettant l'étude in vitro de la capacité des molécules ou compositions dont doit être testée la capacité de fournir la prolifération de virus du SIDA dans un hôte vivant, d'exercer une action à l'égard des cellules infectées.

De ce point l'invention concerne donc aussi un procédé pour l'étude de l'efficacité d'un tel principe actif, ce procédé comprenant la mise en contact des cellules T-cytotoxiques ayant une spécificité HIV et restreinte par HLA-A2 avec des cellules d'une lignée de cellules tumorales transformées de façon stable avec un gène HLA-A2, l'incubation du mélange desdites cellules en présence à la fois dudit principe actif et la détection de l'éventuelle action lytique dudit principe actif à l'égard desdites cellules tumorales, cette action lytique étant alors comparée à celle induite par un peptide conforme à l'invention, dans des conditions expérimentales semblables. Selon que le principe actif exerce une activité lytique plus ou moins importante que celle du peptide choisi, il pourra en être déduit que la substance à l'étude a une action immunogène T plus ou moins importante que celle du peptide choisi.

Dans ce qui précède, l'invention a été illustrée essentiellement en rapport avec des séquences de peptides issus d'une nucléoprotéine de HIV-1. Il va de soi que l'invention ne saurait être limitée à ce cas de figure 1 et qu'elle pourrait être mise en oeuvre dans des conditions semblables vis-à-vis des nucléoprotéines d'autres rétrovirus, par exemple HIV-2. On conçoit d'ailleurs que la comparaison des séquences peptidiques reconnues comme susceptibles d'avoir une action immunogène du type T dans divers types de rétrovirus HIV peut conduire à l'élaboration d'un peptide commun susceptible d'exercer une action inhibitrice de la prolifération d'une infection due à un rétrovirus HIV, quelle qu'en soit la variante concernée. D'ores et déjà, il peut être envisagé de réunir dans un même peptide des séquences peptidiques répondant aux conditions qui ont été évoquées plus haut et qui contiendraient des sites respectivement spécifiques de plusieurs types de rétrovirus, par exemple HIV-1 et HIV-2.

De préférence les peptides concernés ne contiendront pas plus d'acides aminés qu'il n'en faut pour exercer les actions recherchées.

Il va de soi que l'invention est applicable à la recherche de principes permettant d'inhiber la prolifération d'autres virus à caractère pathogène. A cet égard, on mentionnera à titre d'exemple le virus de la grippe, dans lequel les épitopes ayant une activité T immunogène pourraient être recherchés en mettant en oeuvre dans les essais visant à identifier les séquences T immunogènes spécifiques la nucléoprotéine du virus de la grippe. A cet égard on rappelle les expériences de Townsend et al ((16) et (42)) qui avaient déjà montré expressément l'interaction possible de nucléoprotéines de la grippe et de cellules T-cytotoxiques (CTL). Ces auteurs avaient déjà montré que des fibroblastes "naïfs" transfectés par le gène viral codant pour cette protéine nucléaire sont des cibles pour certains clones de CTL.

La bibliographie jointe concerne des publications auxquelles l'homme du métier peut éventuellement se reporter, dès lors que la présente description ne lui apporte peut-être pas certains des principes antérieurs, sur la base desquels la présente invention a été élaboree. En tant que tels ces articles font partie de la présente description.

### BIBLIOGRAPHIE

1. Kourilsky, P. & Claverie, J-M. Ann. Inst. Pasteur/Immunol. 137D, pp3-21 (1986).
2. Claverie, J-M. & Kourilsky, P. Ann. Inst. Pasteur/Immunol. 137D, pp.425-442 (1986).
3. Kourilsky, P., Chaouat, G., Rabourdin-Combe, & Claverie, J.M. Proc. Natl.. Acad. Sci. USA 84, pp3400-3404 (1987).
4. Germain, R. Nature 322, pp687-689 (1986).
5. Schwartz, R.H. Fundamental Immunology, eds. Paul, W. (Raven Press, New York, 1984), pp.379-438.
6. Unanue, E.R. Annu. Rev. Immunol. 2, pp395-428 (1984).
7. Katz, D.H., Hamaoka, T. & Benacerraf, B. J. Exp. Med. 137, pp1405-1418 (1973).
8. Zinkernagel, R.M. & Doherty, P.C. Nature 248, pp701-702 (1974).
9. Shearer, G.M., Rhen, T.G. & Garbarino, C.A. J. Exp. Med. 141, pp1348-1364 (1975).
10. Waldron, J.A., Horn, R.G. & Rosenthal, A.S. J. Immunol 111, pp58-64 (1973).
11. Babbitt B. Allen, P., Matsueda, G., Haber E. & Unanue, E. Nature 317, pp359-361 (1985).
12. Buus, J., Sette, A., Colon, S., Jenis, D. & Grey, H. Cell 47, pp1071-1077 (1986).
13. Buus J., Sette, A., Colon, S., Miles C. & Grey, H. Science 235, pp1353-1358 (1987).
14. Guillet, J., Lai, M., Briner, J. Smith, J. & Gefter, M. Nature 324, pp260-262 (1986).
15. Guillet, J. et al., Science 235, pp865-870 (1987).
16. Townsend, A. et al., Cell 44, pp959-968 (1986).
17. McMichael, A., Gotch, F. & Rothbard, J. J. Exp. Med. 164, pp1397-1406 (1986).
18. Maryanski, J.L., Pala,P. Corradin, G., Jordan, B.R. & Cerottini, J-C. Nature 324, 578-579 (1986).
19. Maryanski, J-L., Abastado, J-P. & Kourilsky, P. Nature 330, pp660-662 (1987).
20. Bjorkman, P.J. et al. Nature 329, pp506-512 (1987).
21. Bjorkman, P.J. et al. Nature 329, pp512-518 (1987).
22. DeLisi, C. & Berzofsky, J. Proc. Natl. Acad. Sci. USA 82, pp7048-7053 (1986).
23. Spouge, J. et al., J. Immunol. 138, pp204-212 (1987).
24. Rothbard, J.B. & Taylor, W.R. EMBO J. 7, pp93-100 (1988).
25. Chalufour, A., Bougueleret, L., Claverie, J-M. & Kourilsky, P. Ann. Inst. Pasteur/Immunol. 138, 671-685 (1987).
26. Alizon, M., Wain-Hobson, S., Montagnier, L., et Sonigo, P. Cell, 46, pp63-74 (1986).
27. Plata, F. et al., Nature, 328, pp348-351 (1987).
28. George, D.G., Barker, W.C. & Hunt, L.T. Nucleic Acids Res. 14, pp11-15 (1986).
29. Claverie, J-L. & Bougueleret, L. Nucleic Acids Res. 14, pp179-196 (1986).
30. Siegel, S. Nonparametric statistics for the behavioural Sciences, (Mac Graw-Hill Eds, New York, 1956).
31. Koller, T.D. Clayberger C., Maryanski, J-L. et Krensky A.M. J. Immunol. 138, pp2044-2049 (1987).
32. Plata, F., and Lilly. F. J. Exp. Med. 150, pp1174-1186 (1979).
33. Robson, B. & Garnier, J., Introduction to Proteins and Protein Engineering. (Elsevier, New York, 1986) pp.347-416.
34. Walker, B.D., et al., Nature, 328, pp345-348 (1987).
35. Sela, M. Science 166, pp1365-1374 (1969).
36. McDevitt, H.O. & Benacerraf, B. Adv. Immunol. 11, pp31-74 (1969).
37. Silman, H.I. & Sela, M. Poly-alpha amino acids eds. Fasman, G.D. (Dekker, New York, 1967) p605.
38. Borek, F., Stupp, Y. & Sela, M. Science 150, pp1177-1178 (1965).
39. Allen, P.M., et al., Nature 327, pp713-715 (1987).
40. Sette, A., et al., Nature 328, pp395-399 (1987).
41. Sela, M., Schechter, B. Schechter, I. & Borek, F. Cold Spring Harbor Symp. Quant. Biol. 32, pp537-545 (1967).
42. Townsend, A. et al, Cell 1986; 44:959-968.

## Revendications

1. Peptide contenant une séquence en acides aminés en commun avec une nucléoprotéine de virus notamment de rétrovirus, contre laquelle une protection chez un hôte supérieur susceptible d'être infecté par ce virus est recherchée, dont les protéines qui lui sont propres peuvent être répertoriées dans un catalogue de séquences tétrapeptidiques caractéristiques du "soi somatique" ou du "soi immunologique", ce peptide étant lui-même caractérisé par le fait qu'il comporte x acides aminés et que les (x-3) séquences tétrapeptidiques successives entrant dans sa constitution, qui se chevauchent mutuellement, et qui sont respectivement décalées d'un acide aminé les unes par rapport aux autres, ne sont pas elles-mêmes, pour l'essentiel, contenues ou susceptibles d'être répertoriées dans le susdit catalogue, ledit peptide ayant la propriété de faire obstacle à la capacité du virus à proliférer dans des cultures cellulaires sensibles à l'infection par ce virus.

2. Peptide selon la revendication 1, caractérisé en ce que la susdite nucléoprotéine correspond à une nucléoprotéine gag de HIV.

3. Peptide selon la revendication 2 contenant une séquence tétrapeptidique contenue dans l'un des peptides suivants :
GNFLQSRPEPTAPPA (gag 3)
GHQAAMEMLKE (gag 5)
HAGPIAPGQMREPRG (H15G)
KEGHQMKDCTERQANF (K16F)
étant entendu que chacun des autres aminoacides contenus dans les séquences qui, le cas échéant, jouxtent ladite séquence tétrapeptidique sont tels qu'essentiellement ils ne forment pas avec les aminoacides voisins des séquences tétrapeptidiques elles-mêmes, pour l'essentiel, contenues, ou susceptibles d'être répertoriées dans un catalogue de séquences tétrapeptidiques, appartenant elles-mêmes à des protéines caractéristiques du "soi somatique" ou du "soi immunologique" de l'homme.

4. Peptide selon l'une quelconque des revendications 1 à 3 caractérisé en ce qu'il contient au plus 100 aminocides .

5. Peptide selon la revendication 4, caractérisé en ce qu'il est constitué d'un hexapeptide.

6. Peptide selon la revendications 4, caractérisé en ce qu'il comprend de 8 à 20 aminoacides.

7. Peptide selon la revendication 4, caractérisé en ce qu'il comprend de 8 à 10 aminocides.

8. Peptide selon la revendication 3, ayant la formule :
K16F : KEGHQMKDCTERQANF

9. Peptide selon la revendication 3 , ayant la formule :
H15G : HAGPIAPGQMREPRG.

10. Peptide selon la revendication 3 , ayant la formule :
gag 5 : GHQAAMEMLKE.

11. Peptide selon la revendication 3 , ayant la formule :
gag 3 : GNFLQSRPEPTAPPA.

12. Procédé pour l'étude de l'efficacité d'un principe actif de médicament antiviral à l'égard d'une infection par un virus de cellules sensibles comprenant :
- la mise en contact de cellules T-cytotoxiques ayant une spécificité liée à ce virus et restreinte par HLA-A2 avec des cellules d'une lignée de cellules tumorales transformées de façon stable avec un gène HLA-A2 et
- l'incubation du mélange de cellules en présence dudit principe actif,
caractérisé par le fait que l'on compare l'action lytique dudit principe actif à l'égard desdites cellules tumorales à l'action lytique obtenue dans les mêmes conditions, en présence dudit peptide selon l'une quelconque des revendications 1 à 10.

13. Procédé selon la revendication 11 pour l'étude in vitro de l'efficacité d'un principe ctif de médicament contre une infection par HIV, caractérisé en ce que les cellules T cytotoxiques mises en oeuvre ont une spécificité HIV.

14. Composition pour provoquer la lyse et la destruction de cellules infectées par un virus, notamment un rétrovirus HIV, caractérisée en ce qu'elle contient le peptide selon l'une quelconque des revendications 1 à 11 , en une dose efficace.

15. Composition selon la revendication 14, caractérisée en ce qu'elle est formée par une association dudit peptide avec un véhicule pharmaceutiquement acceptable.

## Claims

1. Peptide containing an amino acid sequence in common with a virus, especially a retrovirus, nucleoprotein, against which protection in a higher host capable of being infected by this virus is sought, whose proteins which are specific to it may be listed in a catalogue of tetrapeptide sequences characteristic of the "somatic self" or of the "immunological self", this peptide being itself characterized in that it contains x amino acids and in that the (x-3) successive tetrapeptide sequences entering into its constitution, which overlap mutually, and which are respectively spaced one amino acid apart relative to each other, are not themselves, in essence, contained or capable of being listed in the abovementioned catalogue, the said peptide having the property of blocking the capacity of the virus to proliferate in cell cultures sensitive to infection by this virus.

2. Peptide according to Claim 1, characterized in that the abovementioned nucleoprotein corresponds to a HIV gag nucleoprotein.

3. Peptide according to Claim 2, containing a tetrapeptide sequence contained in one of the following peptides:
GNFLQSRPEPTAPPA (gag 3)
GHQAAMEMLKE (gag 5)
HAGPIAPGQMREPRG (H15G)
KEGHQMKDCTERQANF (K16F)
it being understood that each of the other amino acids contained in the sequences which are, where appropriate, next to the said tetrapeptide sequence are such that, essentially, they do not form with the neighbouring amino acids tetrapeptide sequences which are themselves, in essence, contained, or capable of being listed in a catalogue of tetrapeptide sequences, themselves belonging to proteins characteristic of the "somatic self" or of the "immunological self" in man.

4. Peptide according to any one of Claims 1 to 3, characterized in that it contains at most 100 amino acids.

5. Peptide according to Claim 4, characterized in that it consists of a hexapeptide.

6. Peptide according to Claim 4, characterized in that it comprises from 8 to 20 amino acids.

7. Peptide according to Claim 4, characterized in that it comprises from 8 to 10 amino acids.

8. Peptide according to Claim 3, having the formula:
K16F : KEGHQMKDCTERQANF.

9. Peptide according to Claim 3, having the formula:
H15G : HAGPIAPGQMREPRG.

10. Peptide according to Claim 3, having the formula:
gag 5 : GHQAAMEMLKE.

11. Peptide according to Claim 3, having the formula:
gag 3 : GNFLQSRPEPTAPPA.

12. Process for studying the efficacy of an antiviral drug active ingredient against an infection, by a virus, of sensitive cells comprising:
- bringing cytotoxic T cells having a specificity linked to this virus and restricted by HLA-A2 into contact with cells of a tumour cell line stably transformed with an HLA-A2 gene and
- incubating the cell mixture in the presence of the said active ingredient, characterized in that the lytic action of the said active ingredient against the said tumour cells is compared to the lytic action obtained under the same conditions, in the presence of the said peptide according to any one of Claims 1 to 10.

13. Process according to Claim 11, for the in vitro study of the efficacy of a drug active ingredient against an HIV infection, characterized in that the cytotoxic T cells used have an HIV specificity.

14. Composition for causing the lysis and the destruction of cells infected with a virus, especially an HIV retrovirus, characterized in that it contains the peptide according to any one of Claims 1 to 11, in an effective dose.

15. Composition according to Claim 14, characterized in that it is formed by a combination of the said peptide with a pharmaceutically acceptable vehicle.

## Patentansprüche

1. Peptid, welches eine mit einem Nukleoprotein eines Viruses, insbesondere eines Retroviruses, gemeinsame Aminosäuresequenz enthält, gegen das ein Schutz bei einem höherstehenden Wirt, der von diesem Virus infiziert werden kann, gesucht wird, dessen ihm eigene Proteine in einem Katalog aus Tetrapeptid-Sequenzen aufgezeichnet werden können, die für das "somatische Ich" oder das "immunologische Ich" charakteristisch sind, wobei das Peptid seinerseits dadurch gekennzeichnet ist, daß es x Aminosäuren aufweist und daß die (x - 3) in seinen Aufbau miteingehenden aufeinanderfolgenden Tetrapeptid-Sequenzen, die sich gegenseitig überlappen und die jeweils um eine Aminosäure zueinander versetzt sind, ihrerseits in dein Katalog im wesentlichen nicht enthalten sind oder in ihm aufgezeichnet werden können, wobei das Peptid die Eigenschaft hat, der Fähigkeit des Viruses zur Vermehrung in Zellkulturen entgegenzuwirken, welche von diesem Virus infiziert werden können.

2. Peptid nach Anspruch 1, dadurch gekennzeichnet, daß das oben genannte Nukleoprotein einem gag-Nukleoprotein von HIV entspricht.

3. Peptid nach Anspruch 2, das eine Tetrapeptid-Sequenz enthält, die in einem der folgenden Peptide enthalten ist:
GNFLQSRPEPTAPPA (gag 3)
GHQAAMEMLKE (gag 5)
HAGPIAPGQMREPRG (H15G)
KEGHQMKDCTERQANF (K16F)
wobei jede der in den Sequenzen enthaltenen weiteren Aminosäuren, die gegebenenfalls an die Tetrapeptid-Sequenz angrenzen, dergestalt sind, daß sie im wesentlichen mit den benachbarten Aminosäuren keine eigenen Tetrapeptid-Sequenzen bilden, die im wesentlichen in einem Katalog von Tetrapeptid-Sequenzen enthalten sind, oder in ihm aufgezeichnet werden können, welche ihrerseits Proteinen angehören, die für das "somatische Ich" oder das "immunologische Ich" des Menschen charakteristisch sind.

4. Peptid nach einem der Ansprüche 1 bis 3, dadurch gekennzeichnet, daß es höchstens 100 Aminosäuren enthält.

5. Peptid nach Anspruch 4, dadurch gekennzeichnet, daß es aus einem Hexapeptid besteht.

6. Peptid nach Anspruch 4, dadurch gekennzeichnet, daß es 8 bis 20 Aminosäuren aufweist.

7. Peptid nach Anspruch 4, dadurch gekennzeichnet, daß es 8 bis 10 Aminosäuren aufweist.

8. Peptid nach Anspruch 3, welches die folgende Formel hat:
K16F : KEGHQMKDCTERQANF.

9. Peptid nach Anspruch 3, welches die folgende Formel hat:
H15G : HAGPIAPGQMREPRG.

10. Peptid nach Anspruch 3, welches die folgende Formel hat:
gag 5 : GHQAAMEMLKE.

11. Peptid nach Anspruch 3, welches die folgende Formel hat:
gag 3 : GNFLQSRPEPTAPPA.

12. Verfahren zur Untersuchung der Wirksamkeit eines Wirkstoffs eines antiviralen Medikaments hinsichtlich einer Virusinfektion empfindlicher Zellen, mit den folgenden Schritten:
- Zusammenbringen von zytotoxischen T-Zellen, die eine auf diesen Virus beschränkte und durch HLA-A2 eingeschränkte Spezifizität haben, mit Zellen einer Linie von Tumorzellen, die mit einem HLA-A2-Gen stabil transformiert wurden, und
- Inkubation des Zellgemisches in Gegenwart des Wirkstoffes, gekennzeichnet durch die Tatsache, daß man die lytische Wirkung des Wirkstoffs gegenüber den Tumorzellen mit der lytischen Wirkung vergleicht, die man unter den gleichen Bedingungen in Gegenwart des Peptids nach einem der Ansprüche 1 bis 10 erhält.

13. Verfahren nach Anspruch 12 zur in-vitro-Untersuchung der Wirksamkeit eines Medikamentenwirkstoffs gegen eine HIV-Infektion, dadurch gekennzeichnet, daß die verwendeten zytotoxischen T-Zellen eine HIV-Spezifizität haben.

14. Zusammensetzung zur Bewirkung der Lyse und der Zerstörung von Zellen, die durch einen Virus, insbesondere einen HIV-Retrovirus, infiziert sind, dadurch gekennzeichnet, daß sie das Peptid nach einem der Ansprüche 1 bis 11 in einer wirkungsvollen Dosis enthält.

15. Zusammensetzung nach Anspruch 14, dadurch gekennzeichnet, daß sie aus einem Zusammenschluß des Peptids mit einem akzeptablen pharmazeutischen Träger gebildet ist.
